# EUROPEAN PATENT APPLICATION

(11) **EP 1 795 198 A1**
(43) Date of publication of application: **13.06.2007**
(21) Application number: 05077832.3
(22) Date of filing: 09.12.2005
(51) Int. Cl.: A61K 31/551, A61K 31/55, A61K 31/222, A61P 35/00

(54) **Treatment of Barret's esophagus**

(71) Applicant: Hubrecht Laboratorium, 3584 CT Utrecht (NL)
(72) Inventor: Clevers, Johannes Carolus, 3712 AP Huis ter Heide (NL); Van Es, Johannes Hendrikus, 3601 CM Maarssen (NL)
(74) Representative: Winckels, Johannes Hubertus F.

(57) **Abstract**

The invention relates to the field of biochemistry and medicine. More specifically, the invention relates to the treatment of epithelial changes in the esophagus. Even more specific, the invention relates to the treatment of Barrett's esophagus. The invention provides a method for at least in part decreasing Barrett's esophagus present in an animal comprising at least in part inhibiting Notch pathway activation in said animal. Preferably, said inhibition of Notch pathway activation is accomplished via a γ-secretase inhibitor.
Active compounds are DATP, LY-411575, dibenzazepine and benzodiazepine.

## Description

The invention relates to the field of biochemistry and medicine. More specifically, the invention relates to the treatment of epithelial changes in the esophagus. Even more specific, the invention relates to the treatment of Barrett's esophagus.

Barrett's esophagus is a human disease in which the epithelial lining of the esophagus changes from its normal squamous form to an epithelium that resembles that found in the intestine. Barrett's esophagus is a precancerous condition, which predominantly affects white males over 50 years although others may also have this condition.

The epithelial changes result from chronic regurgitation (reflux) of acidic stomach contents back into the esophagus. The contents of the stomach contain digestive enzymes and acid that damage the squamous epithelium of the esophagus. In the healing process, abnormal cells grow back to repair the lesions. This histopathological phenomenon is termed "metaplasia". Cells that resemble intestinal epithelium replace the normal squamous type cells that line the esophagus, a phenomenon termed intestinal metaplasia. This happens in about 10-15 % of people that have long-term gastric reflux.

The metaplastic epithelium may resemble intestinal epithelium but is not normal because it resides at the wrong anatomical site to be considered intestinal epithelium. Moreover, the metaplastic epithelium has a high tendency to become malignant. Patients with Barrett's esophagus have a 30-125 fold higher risk of developing cancer of the esophagus than the general population.

Cancer in Barrett's esophagus develops in a sequence of changes, from nondysplastic (metaplastic) columnar epithelium, through low-grade and then high-grade dysplasia and finally invasive cancer.

The diagnosis of Barrett's disease is typically performed as follows. Individuals who have had reflux symptoms (usually heartburn) for several years undergo upper endoscopy to determine if Barrett's esophagus is present and to assess for premalignant features. This allows the assessment of inflammation (esophagitis) and Barrett's esophagus. The diagnosis of Barrett's esophagus is made by tissue biopsy and subsequent histological analysis. Endoscopists may use special non-toxic dyes such as methylene blue to identify areas with abnormal precancerous changes (dysplasia). This technique is called "chromoendoscopy".

In the absence of dysplasia, patients are kept under surveillance and the acid reflux is treated. When dysplasia is present a more aggressive strategy is usually followed, which involves the destruction of the dysplastic lesions by surgery, by thermal, chemical or mechanical endoscopic techniques. No specific drugs exist that can eradicate the metaplastic or dysplastic epithelium while leaving the squamous epithelium untouched.

Examples of current treatment include surgical treatment of high-grade dysplasia, endoscopic ablation therapy for removal of high-grade dysplasia in the esophagus, chemical ablation via photodynamic therapy for dysplasia, thermal ablation, mechanical ablation or combination therapy.

As already mentioned above, there are at present no specific drugs that can eradicate the metaplastic or dysplastic epithelium in Barrett's esophagus.

The present invention solves this problem by providing a drug that is capable of at least in part removing metaplastic or dysplastic epithelium in Barrett's esophagus. Preferably, said drug does not have a (visible) negative effect on the (underlying) squamous epithelium.

In a first embodiment the invention provides a method for at least in part decreasing Barrett's esophagus present in an animal comprising at least in part inhibiting Notch pathway activation in said animal.

Notch signalling controls spatial patterning and cell fate decisions throughout the animal kingdom (Artavanis-Tsakonas et al 1999). The Notch genes encode large, single transmembrane receptors. Interaction between Notch receptors and ligands results in proteolytic cleavages of the receptor. The resulting free Notch intracellular domain (NICD) translocates into the nucleus, where it binds to the transcription factor RBP-Jκ (CSL or CBF1), thus activating target gene transcription (Baron 2003, Mumm & Kopan 2000). The best-characterized Notch target genes are the hairy/enhancer of split (HES) transcriptional repressors. The HES proteins in turn repress expression of downstream genes (Heitzler et al 1996, Oellers et al 1994).

While Notch pathway components are expressed in mouse intestine (Schroder & Gossler 2002), genetic evidence for the involvement of these components in the control of epithelial cell fates is currently not available. Animals deficient in HES-1, known to represent a Notch target gene in other tissues, show a relative increase in mucosecreting and enteroendocrine cells at the expense of adsorptive cells (Jensen et al 2000). A putative downstream target of HES-1 repression in the intestine, Math-1 (Jensen et al 2000) (Zheng et al 2000) is required for commitment towards the secretory linage as revealed by gene knockout (Yang et al 2001). These results have been interpreted to indicate that Notch signalling skews the fate of differentiating crypt cells leaving the transit amplifying compartment towards an enterocyte phenotype. In this scheme, Notch signalling activates transcription factor genes such as HES1, which in turn repress genes like Math1, driving differentiating cells away from the secretory lineage.

Indirect support for the control of intestinal cell fate by Notch stems from the use of gamma-secretase inhibitors, as originally developed for Alzheimer's disease. Notch is one of several known γ-secretase substrates. Proteolytic processing of Notch by γ-secretase is an essential step following activation of the pathway. As a consequence, one of the effects of γ-secretase inhibitors is the abrogation of Notch pathway activation (De Strooper et al 1999, Kopan & Goate 2000). Rodent toxicological studies with these inhibitors have revealed increases in size and number of mucosecreting goblet cells (Searfoss et al 2003; Wong et al 2004; Milano et al, 2004). Due to these kinds of studies multiple promising γ-secretase inhibitors have been stopped in their further clinical development for the treatment of Alzheimer, because they are heavily suspected of inducing intestinal abnormalities.

The invention discloses the surprising finding that inhibitors of Notch pathway activation (for example γ-secretase inhibitors) are extremely useful in the treatment of Barrett's esophagus. Treatment of Barrett's esophagus with an inhibitor of Notch pathway activation results in the (at least partial) clearance of the metaplastic or dysplastic epithelium from the esophagus.

Notch pathway activation typically goes along the following events. Notch is a transmembrane surface receptor that can be activated through multiple proteolytic cleavages, one of them being cleavage by a complex of proteins with protease activity, termed γ-secretase. Gamma (γ)-secretase is a protease that performs its cleavage activity within the membrane. Gamma (γ)-secretase is a multicomponent enzyme and is composed of at least four different proteins, namely, presenilins (presenilin 1 or 2), nicastrin, PEN-2 and APH-1. Presenilin is the catalytic centre of γ-secretase. On ligand binding the Notch receptor undergoes a conformational change that allows ectodomain shedding through the action of an ADAM protease which is a metalloprotease. This is followed immediately by the action of the γ-secretase complex which results in the release of the Notch intracellular domain (NICD). NICD translocates to the nucleus where it interacts with CSL (C-promoter-binding factor/recombinant signal-sequence binding protein Jκ/Supressor-of-Hairless/Lag1). The binding of NICD converts CSL from a transcriptional repressor to an activator which results in the expression of Notch target genes.

The present inventors describe a method that through inhibition of the Notch pathway activation in an animal suffering from Barrett's esophagus results in at least a partial decrease of metaplastic and/or dysplastic epithelium in said animal.

By providing an inhibitor of Notch pathway activation, the proliferative capacity of metaplastic and/or dysplastic epithelium in the esophagus is at least in part decreased. Preferably, the decrease is such that it is visible with an endoscope or an exploratory operation followed by histological analysis. Even more preferably, the decrease of metaplastic and/or dysplastic epithelium is complete, i.e. no (visible) remaining metaplastic and/or dysplastic epithelial cells. The decrease of metaplastic and/or dysplastic epithelium is the result of the conversion of metaplastic and/or dysplastic cells to postmitotic cells (for example goblet cells). These postmitotic cells have a relative short lifespan (5-6 days) and the body clears them after their death.

An animal is herein defined as a non-human animal or a human being (i.e. a human patient or a human suffering from Barrett's disease).

Inhibition (of at least part, but preferably completely) of Notch pathway activation is accomplished in different ways, which are (non-limiting) outlined here under. Preferably the inhibition is performed locally, i.e. in the esophagus. The inventors have demonstrated in the experimental part that interference with the Notch pathway in the normal (non-dysplastic, non-metaplastic) squamous epithelium of the esophagus has no detectable effect on that epithelium.

Moreover, Notch pathway activation is defined to include pathway activation of Notch-like molecules and/or different allelic variants of Notch (like) molecules.

Barrett's esophagus is herein defined as not only to comprise the metaplastic epithelium but also (if present) the dysplastic epithelium. Hence, the invention is used for the treatment of the non-tumorigenic phases as well as the cancerous phases.

In a preferred embodiment the invention provides a method for at least in part decreasing Barrett's esophagus present in an animal comprising at least in part inhibiting Notch pathway activation in said animal wherein said Notch pathway activation is at least in part inhibited by providing said animal with a γ-secretase inhibitor. Such a γ-secretase inhibitor is for example peptidic in nature or non-peptidic or semi-peptidic and is preferably a small molecule. Gamma-secretase inhibitors were originally defined for Alzheimer's disease. A key step in the pathogenesis of Alzheimer's disease is APP proteolysis resulting in the formation of the amyloid-β peptide (Aβ), the principle protein component of the characteristic cerebral plaques of the disease. APP (just like Notch) is first cleaved in the extracellular domain (in this case by a β-secretase) and the remaining part of APP is cleaved within the membrane by γ-secretase to produce Aβ peptide. Inhibition of Aβ peptide production by blocking γ-secretase activity is at present one of the most promising therapeutic strategies to slow progression of Alzheimer's disease pathology. Several companies have in the meantime developed γ-secretase inhibitors, such as DAPT (N-[N-(3,5-difluorophenylacetyl)-L-alanyl]-S-phenylglycine t-butyl ester). Also compounds from the chemical classes AS (arylsulfonamide), DBZ (dibenzazepine (DBZ), BZ (benzodiazepine), LY-411,575 and many others, have been tested for their γ-secretase inhibiting activity. An overview in respect of γ-secretase inhibitors is for example outlined in Harrison et al 2004 in which the γ-secretase inhibitors have been divided in solfonamides/sulfones and benzodiazepines/benzolactams. Several of these γ-secretase inhibitors have already been in clinical phase I and II trials. It is clear to a skilled person that a method for at least in part decreasing Barrett's esophagus present in an animal comprising at least in part inhibiting Notch pathway activation in said animal wherein said Notch pathway activation is at least in part inhibited by providing said animal with an inhibitor of y-secretase, can be performed by providing at least one or at least two or more γ-secretase inhibitors, i.e. by providing a combination of different γ-secretase inhibitors. It is clear that γ-secretases are typically capable of acting in (at least) two pathways: the APP and the Notch pathway. Chemical companies have in the meantime developed γ-secretases which are specific for one or the other, e.g. γ-secretases that are specific for the APP pathway and do not interfere with the Notch pathway. It is clear that γ-secretases that do not interfere with the Notch pathway are not useful in a method of the invention. Hence, preferably a γ-secretase capable of interfering in the Notch pathway as well as in the APP pathway or γ-secretases capable of specifically interfering in the Notch pathway are preferred. Such inhibitors can for example be found in Harrison et al 2004, which are incorporated herein by reference.

In a preferred embodiment the invention provides a method for at least in part decreasing Barrett's esophagus present in an animal comprising at least in part inhibiting Notch pathway activation in said animal wherein said Notch pathway activation is at least in part inhibited by providing said animal with a γ-secretase inhibitor, wherein said inhibitor of γ-secretase is DAPT or dibenzazepine (DBZ) or benzodiazepine (BZ).

As already outlined above γ-secretase is a complex of proteins. Another way of at least in part inhibiting Notch pathway activation is accomplished by at least in part inhibiting the formation of said complex of proteins because only the complex is considered to be active. This is for example accomplished by providing one of the components as a dominant negative molecule or by providing a part/molecule of said complex which part/molecule comprises a mutation preventing further complex formation or resulting in an unstable (non-active) protein complex. Yet another way of at least in part inhibiting Notch pathway activation is by specifically inhibiting the catalytic part of said complex, i.e. specific inhibition of the presenilins.

In another preferred embodiment the invention provides a method for at least in part decreasing Barrett's esophagus present in an animal comprising at least in part inhibiting Notch pathway activation in said animal, wherein said Notch pathway activation is at least in part inhibited by at least in part diminishing ligand mediated activation of Notch. As outlined above, Notch pathway activation starts with ligand binding after which event the Notch receptor undergoes a conformational change that allows ectodomain shedding through the action of an ADAM protease. It is clear to a skilled person that the ligand binding to Notch may be at least partly, but preferably completely, inhibited by multiple strategies. Preferably said ligand-mediated activation of Notch is at least in part diminished by providing said animal with a dominant-negative ligand of Notch. Examples of natural Notch ligands are the proteins Delta, Jagged and Serrate. Dominant negative ligands, i.e. ligands capable of binding to Notch essentially without further activation of Notch pathway (blocking of Notch pathway activation), may be derived from said natural ligands, for example by producing small binding molecules based upon the binding part of said natural ligand. In case such dominant negative ligands are brought into contact with Notch, binding of said dominant negative ligand to Notch takes place without further activation of the Notch pathway. Preferably, said dominant negative ligand sticks/binds for longer periods of time to Notch and binding of natural ligand is partly and preferably completely blocked/inhibited and as a consequence Notch pathway activation (is at least in part) inhibited. Examples of dominant negative ligand are for example mutants of Delta and Serrate comprising intracellular deletions (Sun and Artavanis-Tsakonas, 1996).

In another preferred embodiment said ligand mediated activation of Notch is at least in part diminished by providing said animal with a dominant negative Notch. In principle each type of Notch molecule may be used for this purpose, i.e. Notch1, 2, 3 or 4 or a functional fragment and/or a functional derivative thereof. A functional fragment is any fragment (N-terminal fragment, C-terminal fragment or an internal fragment or any combination thereof) derived from either of these molecules (or equivalent thereof) which is capable of binding to Notch ligand. Such a functional fragment may for example be present as a membrane or as a non-membrane bound compound. By binding of the dominant negative Notch to the available ligands, said ligand cannot bind to naturally/originally/functionally available Notch and hence Notch pathway activation is at least in part inhibited. A functional derivative is for example a Notch molecule which has been mutated (point mutation, insertions) such that binding to a ligand is still possible but that the mutation prevents the signal of ligand binding to be transmitted. A functional derivative may also be derived from another species. In yet another preferred embodiment said ligand mediated activation of Notch is at least in part diminished by providing an animal in need thereof with an antibody capable of at least in part blocking the interacting between a Notch ligand and Notch. Such an antibody is for example directed to the ligand binding part of Notch or directed to the part of the ligand that interacts with Notch. The production of antibodies is routine within in the art and hence no further details regarding this matter is provided. Independent on the type of used inhibition of ligand mediated activation of Notch, the result is the same: the formation of (at least part of) NICD is inhibited, which eventually results in the clearance of metaplastic and/or dysplastic epithelium from the esophagus.

As already indicated, inhibition (of at least part of, but preferably completely) of Notch pathway activation is accomplished in different ways. In yet another preferred embodiment, the invention provides a method for at least in part decreasing Barrett's esophagus present in an animal comprising at least in part inhibiting Notch pathway activation in said animal, wherein said Notch pathway activation is at least in part inhibited by providing said animal with an ADAM protease inhibitor. After binding of a Notch ligand to Notch, the Notch receptor undergoes a conformational change that allows ectodomain shedding through the action of an ADAM protease. ADAM stands for a disintegrin and metalloprotease. By providing a substance capable of inhibiting the protease that accomplishes the ectodomain shedding, the Notch pathway activation is at least partly but preferably completely inhibited, i.e. no formation of NICD occurs. Again, local inhibition of Notch pathway activation is preferred to avoid, as much as possible, any possible undesired side effects.

It is clear to a person skilled in the art that also different ways of at least in part inhibiting Notch activation may be combined to, for example, increase efficacy.

In a preferred embodiment the invention provides use of a Notch pathway inhibitor in the preparation of a medicament for the treatment of Barrett's esophagus. As already outlined above for the methods according to the invention, multiple inhibitors may be used to at least in part decrease the formation of NICD and hence to at least in part prevent Notch pathway activation. Examples of preferred Notch inhibitors are: γ-secretase inhibitors, such as DAPT or dibenzazepine (DBZ) or benzodiazepine (BZ), an inhibitor capable of diminishing ligand mediated activation of Notch (for example via a dominant negative ligand of Notch or via a dominant negative Notch or via an antibody capable of at least in part blocking the interacting between a Notch ligand and Notch), or an inhibitor of ADAM proteases. Moreover, such an inhibitor may be combined with already available therapy such as chemotherapy, surgery or irradiation.

In yet another embodiment the invention provides a pharmaceutical composition comprising at least two Notch pathway activation inhibitors.

Pharmaceutical compositions according to the invention may be provided in the form of a powder, a solution or suspension in a(n) (non) aqueous liquid or as an emulsion. Said pharmaceutical may further comprise pharmaceutically acceptable excipients, carriers and/or diluents. The pharmaceutical compositions may be administered orally, parenterally, intramuscularly, intravenously, parentally, intraperitonally or colorectally (for example with a suppository).

Oral delivery is preferred because the to be treated areas are easily reached via this route of delivery. Moreover, the delivery is preferably locally to avoid any undesired side effects. Preferably, the Notch pathway activation is at least in part inhibited by providing an animal in need of treatment with for example syrup that sticks to the esophagus. In yet another embodiment, a medicament for at least in part inhibiting Notch pathway activation is provided via endoscopy to make sure that the medicament is locally delivered, i.e delivered at the place that needs treatment. For example delivery of a medicament via injection in the mucosal tissue via an endoscope/gastroscope. In yet another embodiment, a medicament comprising an inhibitor of Notch pathway activation is prepared such that it is destroyed in the stomach after passage through the esophagus. In such a case the medicament is not able to pass the acidic/digestive environment of the stomach without being broken down by the acids and enzymes present in the stomach.

With respect to the doses it is noted that a skilled person is capable, based on for example the already known pharmacokinetics, to determine an effective dosage of for example DAPT and/or DBZ and/or BZ. Moreover, by using one or multiple well-known dose-finding experiments, effective doses can be determined by the skilled person.

Besides the use of a pharmaceutical in any of the outlined methods, a pharmaceutical according to the invention is also very useful as an additive (residual) to other therapies. As outlined herein, current therapies include surgical treatment of high-grade dysplasia, endoscopic ablation therapy for removal of high-grade dysplasia in the esophagus, chemical ablation via photodynamic therapy for dysplasia, thermal ablation, mechanical ablation or combination therapy. It is clear that a treatment according to the present invention can be combined with any of these more traditional methods.

The invention will be explained in more detail in the following description, which is not limiting the invention.

### Experimental part

### Experiment 1

### Material and methods

### Tissue sample preparation, immunohistochemistry

The esophagus was dissected as a whole and flushed gently with Formaline and subsequently fixed in Formalin for 16 hours. The tissues were sectioned (2-6 µm). Following dewaxing and hydration, sections were pretreated with peroxidase blocking buffer (120 mM Na₂HPO₄, 43 mM citric acid, 30 mM NaN₃, 0.2% H₂O₂; pH 5.8) for 15 minutes at room temperature. Antigen retrieval was performed by boiling samples in Na-citrate buffer (10 mM, pH 6.0). After 20 minutes, the boiling pan was allowed to slowly cool down to room temperature.

The following antibodies were used. Mouse anti-Ki67 (1:100; Novocastra), mouse anti-β-catenin (1:50; Transduction Labs), Mouse anti-BrdU (1:500; Becton Dickinson), Rabbit anti-Mathl (1:50; a kind gift of Dr Jane Johnson). Incubation of antibodies was performed in BSA in PBS overnight at 4°C for antibodies directed against Caspase-3 and Math1 and at RT for 1 hour for antibodies directed against Ki67, BrdU, Synaptofysin and β-catenin. In all cases, the Envision⁺ kit (DAKO) was used as a secondary reagent. Stainings were developed using DAB. Slides were counterstained with hematoxylin and mounted.

Goblet cells were stained by Alcian Blue and PAS staining. For Alcian Blue staining of Goblet cells, following dewaxing and hydration, tissues were incubated for 5 min. in Alcian Blue (1% in 0.5% acetic acid). Subsequently washed in water, 1 min. incubated in neutral red. Quickly dehydrated, washed in Xylene and mounted with Pertex. For PAS staining of Goblet cells, following dewaxing and hydration, tissues were oxidize in 0.5% periodic acid solution for 5 min. Subsequently washed in water, 15 min. incubated in Schiff reagent, washed in lukewarm water for 5 min., counterstained in hematoxylin solution for 1 min, washed in water for 5 min., dehydrated and mounted.

### Pharmacological inhibition of Notch signaling in APC^{min} mice

8-week-old APC^{min} were treated with 2 different, orally deliverable Υ-secretase inhibitors for 10 days, after which the esophagus and the intestines were examined histologically. The administered γ-secretase inhibitors were benzodiazepine (BZ) and dibenzazepine (DBZ) at a concentration of 15 µmol/Kg. DBZ was suspended finely in 0.5% (w/v) Hydoxypropyl Methylcellulose (Methocel E4M) and 0.1% (w/v) Tween 80 in water, while BZ was suspended finely in 6% (v/v) Ethanol/94% (v/v) Labrafil M 1944 CS.

### Results

After the oral treatment, the animals were sacrificed. The entire esophagus and intestines were removed and subjected to histological analysis.

As expected, the animals developed near-complete conversion of proliferative crypt cells into postmitotic goblet cells, as described by van Es et al, Nature 2005. This indicated that the oral doses effectively inhibited gamma-secretase throughout the small intestine. Meanwhile, careful scrutiny of all histological data obtained from the esophagus of the same mice did not reveal any changes in histology and proliferative activity. This shows that under the given conditions the healthy esophagus is unaffected and is effectively insensitive to gamma-secretase inhibition.

### Experiment 2

### Selection of patients

Patients with Barrett's disease were selected based on techniques as outlined in the description herein

### Treatment of patients

As outlined in detail in the description there are multiple suitable routes of administration. Preferably, the administration is locally. Selected patients were treated locally with a γ-secretase inhibitor by injecting said γ-secretase inhibitor via an endoscope/gastroscope in the mucosal tissue of said patients. The used dosage of the γ-secretase inhibitor was dependent on the used specific γ-secretase inhibitor and its pharmokinetics. Moreover, the treatment was repeated as often as necessary (depending on the stage of Barrett's esophagus).

### References

Artavanis-Tsakona S, R and MD, Lake RJ. Notch signalling: cell fate control and signal integration in development. Science. 1999 Apr 30;284(5415):770-6.

Baron M. An overview of the Notch signalling pathway. Semin Cell Dev Biol. 2003 Apr;14(2):113-9.

De Strooper B, Annaert W, Cupers P, Saftig P, Craessaerts K, Mumm JS, Schroeter EH, Schrijvers V, Wolfe MS, Ray WJ, Goate A, Kopan R. Presenilin-1-dependent gamma-secretase-like protease mediates release of Notch intracellular domain. Nature. 1999 Apr 8;398(6727):518-22.

Harrison T. et al. (2004) γ-Secretase as a target for drug intervention in Alzheimer's disease, Current Opinion in Drug Discovery & Development, 7(5); 709-719.

Heitzler P, Bourouis M, Ruel L, Carteret C, Simpson P. Genes of the Enhancer of split and achaete-scute complexes are required for a regulatory loop between Notch and Delta during lateral signalling in Drosophila. Development. 1996 Jan;122(1):161-71.

Jensen J, Pedersen EE, Galante P, Hald J, Heller RS, Ishibashi M, Kageyama R, Guillemot F, Serup P, Madsen OD. Control of endodermal endocrine development by Hes-1. Nat Genet. 2000 Jan;24(1):36-44.

Kopan R, Goate A. A common enzyme connects notch signaling and Alzheimer's disease. Genes Dev. 2000 Nov 15;14(22):2799-806.

Milano J, McKay J, Dagenais C, Foster-Brown L, Pognan F, Gadient R, Jacobs RT, Zacco A, Greenberg B, Ciaccio PJ. Modulation of Notch Processing by {gamma}-Secretase Inhibitors Causes Intestinal Goblet Cell Metaplasia and Induction of Genes Known to Specify Gut Secretory Lineage Differentiation. Toxicol. Sci. 2004 Nov;82(1):341-358.

Mumm JS, Kopan R. Notch signalling: from the outside in. Dev Biol. 2000 Dec 15;228(2):151-65.

Oellers N, Dehio M, Knust E. bHLH proteins encoded by the Enhancer of split complex of Drosophila negatively interfere with transcriptional activation mediated by proneural genes. Mol Gen Genet. 1994 Sep 1;244(5):465-73.

Schroder N and Gossler A. Expression of Notch pathway components in fetal and adult mouse small intestine. Gene Expr Patterns. 2002 Dec;2(3-4):247-50.

Searfoss, G.H. et al (2003) Adipsin, a biomarker of gatrointestinal toxicity mediated by a functional gamma-secretase inhibitor, J.Biol.Chem., Nov 14 (278(46):46107-46116.

Sun, X. and Artavanis-Tsakonas, S. (1996) The intracellular deletions of Delta and Serrate define dominant negative forms of the Drosophila Notch ligands, Development, 122, pages 2465-2474.

Van Es, J.H. et al. (2005) Notch/γ-secretase inhibition turns proliferative cells in intestinal crypts and adenomas into globlet cells, Nature, vol. 435, 16 June 2005, pages 959-963.

Wong GT, Manfra D, Poulet FM, Zhang Q, Josien H, Bara T, Engstrom L, Pinzon-Ortiz M, Fine JS, Lee HJ, Zhang L, Higgins GA, Parker EM. Chronic treatment with the gamma-secretase inhibitor LY-411,575 inhibits beta-amyloid peptide production and alters lymphopoiesis and intestinal cell differentiation. J.Biol Chem 2004 Mar 26;279(13):12876-12882.

Yang Q, Bermingham NA, Finegold MJ, Zoghbi HY. Requirement of Math1 for secretory cell lineage commitment in the mouse intestine. Science. 2001 Dec 7;294(5549):2155-8.

Zheng J.L. et al. (2000) Hes1 is a negative regulator of inner ear hair cell differentiation. Development. Nov;127(21):4551-1560.

## Claims

1. A method for at least in part decreasing Barrett's esophagus present in an animal comprising at least in part inhibiting Notch pathway activation in said animal.

2. A method according to claim 1, wherein said Notch pathway activation is at least in part inhibited by at least in part diminishing ligand mediated activation of Notch.

3. A method according to claim 1 or 2, wherein said Notch pathway activation is at least in part inhibited by providing an inhibitor of γ-secretase.

4. A method according to any one of claims 1 to 3, wherein said inhibitor of γ-secretase is provided as an oral preparation.

5. Use of a Notch pathway inhibitor in the preparation of a medicament for the treatment of Barrett's esophagus.

6. Use according to claim 5, wherein said Notch pathway inhibitor is a γ-secretase inhibitor.

7. Use according to claim 5 or 6, wherein said medicament is an oral medicament.
